# EUROPEAN PATENT SPECIFICATION

(11) **EP 4 171 673 B1**
(45) Date of publication and mention of the grant of the patent: **25.12.2024**
(21) Application number: 21731491.3
(22) Date of filing: 09.06.2021
(51) Int. Cl.: A61L 27/36, A61L 27/54, A61F 2/14, A61K 9/00

(54) **NEW DRUG DELIVERY SYSTEM FOR OPHTALMIC USE**
NEUES ARZNEIMITTELABGABESYSTEM ZUR OPHTHALMISCHEN VERWENDUNG
NOUVEAU SYSTÈME D'ADMINISTRATION DE MÉDICAMENTS POUR USAGE OPHTALMIQUE

(30) Priority: 09.06.2020 EP 20179055
(43) Date of publication of application: 03.05.2023
(73) Proprietor: Dompe' Farmaceutici SpA, 20121 Milano (IT)
(72) Inventor: ACERRA, Giuseppina, 80131 Napoli (IT); DETTA, Nicola, 80131 Napoli (IT); PANDOLFI, Assunta, 66100 Chieti (IT); MASTROPASQUA, Leonardo, 66100 Chieti (IT); NUBILE, Mario, 66100 Chieti (IT); MANDATORI, Domitilla, 66100 Chieti (IT)
(74) Representative: Dompé farmaceutici Spa
(86) International application number: PCT/EP2021/065460
(87) International publication number: WO 2021/250095

(56) References cited:
- WO-A2-2020/008258
- US-A1- 2011 206 773
- US-A1- 2019 307 551

## Description

### Field of the invention

The present invention relates to the field of sustained release ophthalmic formulations for the delivery of active principles to the ocular surface, in particular for the treatment of pathologies of the cornea.

### Background of the invention

Topical instillation of drugs as eye drops is the preferred and conventional route for the treatment of most diseases of the anterior ocular segment.

However, this mode of administration is highly inefficient and characterized by a very poor bioavailability. After instillation, the flow of lacrimal fluid moves the drug from the ocular surface to the nasolacrimal ducts in a few minutes and, thus, the majority of it is lost through the naso-lacrimal drainage. Only 5% of the total amount of drug administered is available for a sufficient period of time in contact with the ocular surface to exert a therapeutic effect.

This requires frequent administration of high amount of drug, with very low patient compliance, and renders effective dosage inaccurate.

Punctal plugs have been used in order to attain effective drug concentration and residence time at the anterior surface of the eye, thus reducing the dose and frequency of administration. These are small medical devices that are inserted into the tear duct of the eye to block the duct and thus prevent the drainage of liquid from the eye. However, they may be not well tolerated by patients and cause complications and such as epiphora, foreign body sensation, infection, pyogenic granuloma, punctal canalicular erosion, dacryocystitis and increased allergy symptoms.

An alternative approach has been the development of sustained release delivery systems that extend corneal residence time of drugs to the anterior segment of the eye, such as viscous solutions, mucoadhesive materials or drug-coated contact lenses.

For example, US20040241207 discloses a contact lens with embedded drug nanoparticles that diffuse through the contact lens into the post-lens tear film when the lens is applied to the eye.

However, contact lenses or other artificial polymers have the inconvenience that they can trigger immune responses in the patients.

In addition to the above, the bioavailability of the drug is further reduced by the physiological barriers of the eye, which greatly limit the drug concentration in the inner layers of the cornea. In particular, the corneal epithelium is lipophilic and presents tight junctions between the cells, which lead to a limited permeation by drug molecules, in particular hydrophilic drug molecules.

US 2019/307551 discloses a drug delivery implant and its method of production; in particular, it discloses a corneal implant associated with a drug releasing implant, which may contain poly(amidoamine) (PAMAM), poly(amidoamine-organosilicon) (PAMAMOS), poly(propyleneimine) (PPIO), poly(caprolactone), poly(lactic acid) (PLA), polylactic-co-glycolic acid (PLGA).

It is therefore felt the need of developing a biocompatible, non-immunogenic delivery system that solves the above problems and allows to obtain sufficient concentrations of drug molecules for a sufficient period of time in the anterior portion of the eye.

### Summary of the invention

The present inventors have surprisingly found that corneal stroma can be functionalized with polylactic co-glycolic acid (PLGA) microparticles containing pharmaceutically active molecules and used for the controlled release delivery of these molecules to the anterior portion of the eye.

Accordingly, a first object of the invention is a drug delivery system suitable for ophthalmic use comprising a decellularized corneal stroma scaffold having dispersed within and/or bound to the surface microparticles containing at least one pharmaceutically active molecule dispersed in a matrix having a composition comprising at least 70% of polylactic co-glycolic acid (PLGA).

A second object of the invention is a process for the manufacturing of a drug delivery system according to the first object of the invention.

A third object of the invention is a pharmaceutically active molecule for use in the treatment of pathologies of the front of the eye, wherein said pharmaceutically active molecule is administered by means of a drug delivery system according to the first object of the invention

Also disclosed but not part of the invention: a method for the treatment of pathologies of the front of the eye, comprising administering a pharmaceutically active molecule by means of a drug delivery system according to the first object of the invention.

### Description of the Figures

Figure 1 represents Transmission Electron Microscopy (TEM) images showing the lenticule with no treatment (panel A) and the lenticule after decellularization with 0.1% SDS (panel B), as described in Example 2.
Figure 2 shows images of lenticules obtained by confocal microscopy, as described in Example 5. In details, panel A shows a 3D reconstruction showing the superficial localization of Fluo-MPs (light grey dots) in the lenticule; panel B shows a 2D image showing the Fluo-MPs distribution (light grey dots) between collagen fibers (dark grey).
Figure 3 shows the vitro kinetic release of rhNGF from lenticule, as measured in Example 6. The chart shows the cumulative release of rhNGF from loaded PLGA-MPs previously included in the lenticule.

### Detailed description of the invention

A first object of the present invention is a drug delivery system comprising a decellularized corneal stroma scaffold having dispersed within and/or bound to the surface microparticles containing at least one pharmaceutically active molecule dispersed in a matrix having a composition comprising at least 70% w/w of polylactic co-glycolic acid (PLGA).

According to a preferred embodiment of the invention, the corneal stroma scaffold according to the first object of the invention is a graft obtained from the cornea of the patient to be treated or from a donor.

For example, said corneal stroma may be obtained from a donor undergoing a refractive eye surgery, preferably refractive lenticule extraction (ReLEx) or femtosecond lenticule extraction (FLEx). Alternatively, the corneal stroma can be of cadaveric donor.

According to another preferred embodiment of the invention, said corneal stroma scaffold is an engineered corneal stroma scaffold obtained in vitro.

Preferably, said corneal stroma scaffold is a corneal stroma lenticule.

Preferably, said corneal stroma lenticule has a thickness between 80 and 300 µm, preferably between 100 and 150 µm and/or a diameter between 4 and 9 mm, preferably between 5 and 8 mm, more preferably between 6 and 7 mm.

Preferably, said corneal stroma scaffold is cell-free.

The absence of cells in the stroma renders it immunogenically compatible and creates pores into the scaffold that incorporate the microparticles.

According to a preferred embodiment, said microparticles are dispersed within the decellularized corneal stroma scaffold.

According to a preferred embodiment, said microparticles are both dispersed within and bound to the surface of the decellularized corneal stroma scaffold.

Preferably, said matrix of the drug delivery system according to the first object of the invention comprises at least 75% (w/w), preferably 80% (w/w), more preferably 85% (w/w) of polylactic co-glycolic acid (PLGA).

Preferably, said matrix comprises between 85% (w/w) and 95% (w/w), more preferably 89% (w/w), of polylactic co-glycolic acid (PLGA).

According to a particularly preferred embodiment, said PLGA has a copolymer composition consisting of between 50% (w/w) and 75 % (w/w), preferably 75% (w/w) of lactic acid and between 50% (w/w) and 25% (w/w), preferably 25% (w/w) of glycolic acid.

According to another preferred embodiment, also in combination with the preceding ones, said PLGA is amorphous.

According to another preferred embodiment, also in combination with the preceding ones, said PLGA has a molecular weight between 4000-15000 g/mol.

A PLGA particularly suitable for use in the drug delivery system of the invention is for example the product commercialized under the trade name Resomer^{®} RG752H, Poly(D,L-lactide-co-glycolide).

Preferably, said matrix further contains, in addition to PLGA, one or more additional components selected from: polyethylene glycol, preferably low-molecular-weight grade polyethylene glycol, more preferably PEG400, albumin, preferably human seric albumin (HSA), and/or Trehalose. Preferably, said matrix has a composition consisting of PLGA, PEG400, HSA and Trehalose, wherein PLGA is present in an amount of at least 70% w/w of the total composition.

According to a preferred embodiment of the invention, said microparticles consist of said matrix with dispersed therein at least one pharmaceutically active molecule.

Preferably, in order to obtain both an effective incorporation in or binding to the corneal stroma scaffold and a suitable release rate of the pharmaceutically active molecule, the microparticles have a particle size distribution measured by laser diffraction technology between 1 and 15 µm, preferably between 1.5 and 10 µm, more preferably between 1.9 and 7 µm. Preferably, said particle size is measured using the particle size analyzer Mastersizer2000 with the Hydro 2000µP wet sample dispersion unit (Malvern Panalytical).

The drug delivery system according to the invention is able to release the pharmaceutically active molecule at a controlled rate, preferably for a period of at least one month.

Preferably, said pharmaceutically active molecule is a protein or a peptide.

Said protein can be of natural origin or synthetically produced, preferably it is a recombinant protein.

According to a preferred embodiment, said pharmaceutically active molecule is a growth factor or a growth-factor mimetic peptide or protein. More preferably, said pharmaceutically active molecule is a neurotrophin or a neurotrophin-mimetic peptide or protein.

According to the present invention the term "neurotrophin" refers to proteins, including recombinant proteins, that act as growth promoters for neurons. These include Nerve Growth Factor (NGF), Brain-Derived Neurotrophic Factor (BDNF), Neurotrophin-3 (NT-3) and Neurotrophin-4 (NT-4); the term "neurotrophin mimetic-peptide or protein" refers to any peptide or protein, of natural or synthetic origin, capable of acting as an agonist for one or more neurotrophin receptors. For example, a neurotrophin mimetic-protein may be a protein that has mutations or truncations with respect to a natural wild type neurotrophin but is able to activate one or more neurotrophin receptors. The mimetic peptides may be either linear or cyclic peptides.

Particularly preferred pharmaceutically active molecules according to the invention are nerve growth factor and nerve growth factor-mimetic peptides or proteins. Said nerve growth factor is preferably human nerve growth factor, more preferably recombinant human nerve growth factor.

The amount of pharmaceutically active molecule dispersed in the microparticle matrix will vary depending on the desired dose to be administered to the anterior part of the eye. Preferably, when said pharmaceutically active molecule is nerve growth factor it is contained in said microparticle matrix in an amount between 30 and 100 ng, preferably between 50 and 90 ng, more preferably between 60 and 80 ng per each mg. According to the present invention, the expression "decellularized corneal stroma scaffold having bound to the surface microparticles" means that the microparticles are hold and retained on the surface of the decellularized corneal stroma scaffold by force of attraction that may be of various kind.

A second object of the present invention is a method for preparing a drug delivery system as described above, comprising the following steps:
a. obtaining a corneal stroma scaffold as above described, preferably a corneal stroma lenticule from a donor cornea as above described;
b. removing cellular material from said scaffold, if present, thus obtaining a decellularized corneal stroma scaffold;
c. incubating said decellularized corneal stroma scaffold in a suspension of microparticles containing at least one pharmaceutically active molecule dispersed in a matrix having a composition comprising at least 70% (w/w) of polylactic co-glycolic acid (PLGA), as described above.

Preferably, in step a. said corneal stroma scaffold is a corneal stroma lenticule with a thickness between 80 and 300 µm, preferably between 100 and 150 µm and/or a diameter between 4 and 9 mm, preferably between 5 and 8 mm, more preferably between 6 and 7 mm.

Preferably, in step b. of the above method according to the invention said cellular material is removed so as to obtain an amount of residual cells in said corneal stroma scaffold lower than 0.5 weight%, more preferably a cell-free lenticule.

Preferably, said cellular material is removed by incubating the lenticule in a solution of a surfactant. According to a preferred embodiment, said surfactant is sodium dodecyl sulfate (SDS), preferably at a concentration between 0.05 and 0.2%, more preferably 0.1%.

In order to obtain an effective removal of the cellular component from the lenticule, the incubation with the solution of surfactant should be carried out for at least 20 hours, preferably for at least 24 hours.

When the corneal stroma scaffold is a corneal stroma lenticule, the decellularized lenticules obtained in step b. may be incubated with the microparticles immediately after extraction from the donor.

More preferably, the decellularized lenticules obtained in step b are dehydrated and stored at room temperature until use. According to this embodiment, the above method further comprises after step b and before step c., a step b'. wherein the decellularized lenticules are dehydrated, preferably by treatment at a temperature between 50 ° and 70 °C, preferably between 55 ° and 65 °C, more preferably at 60 °C for between 1 and 3 hours, preferably 2 hours.

The dehydration of the decellularized lenticules is particularly advantageous since it greatly increases the ability of the microparticles to be incorporated in the stroma. The incubation of the corneal stroma scaffold with the suspension of microparticles in step c. is preferably carried out at room temperature.

Preferably, said incubation is carried out for a period of time between 1 and 5 hours, preferably for 3 hours.

The microparticles of step c. of the method according to the present invention, are obtained by the following steps:
a. preparing a water/oil emulsion containing the pharmaceutically active principle and polylactic co-glycolic acid.
b. preparing an aqueous solution containing polyvinyl alcohol doped with the same organic solvent used in the oil phase of step a.
c. mixing the emulsion and solution prepared in a. and in b. and homogenizing, thus obtaining a water/oil/water emulsion.
d. evaporating the organic solvent, thus obtaining precipitation of microparticles;
e. recovering and washing the precipitated microparticles;
f. freeze-drying the microparticles.

Preferably, in step a. the polylactic co-glycolic acid is present in a concentration between 10 and 30% w/v, preferably between 15 and 25% w/v, more preferably between 18 and 22% w/v.

Preferably, said oil phase in step a. is ethyl acetate.

Preferably, in step b. the concentration of polyvinyl alcohol in the aqueous solution is between 0.5 and 2% w/v, preferably 1% w/v.

The drug delivery system obtained with a process according to the second object of the invention has all the features according to the first object of the invention.

As will be demonstrated in Example 6, the drug delivery device according to the invention is able to release at a controlled rate the pharmaceutically active molecule from the microparticles included in the cornea stroma scaffold for a period of time of at least one month.

The delivery system according to the invention can be advantageously used to deliver pharmaceutically active molecules to the anterior segment of the eye.

The administration to the patient may be effected in different ways, depending on the specific pathology to be treated.

For example, in case it is necessary that the pharmaceutically active molecule exerts its activity on the inner layers of the cornea, the delivery system may be implanted into a surgically created intrastromal pocket in the eye of the patient, for example formed by means of a femtosecond laser. Alternatively, the delivery system may be applied on the surface of the cornea.

The active molecule in the delivery system will be released at a constant rate, thus maintaining an effective concentration at the site of action throughout the whole treatment.

The delivery system according to the invention is highly biocompatible and non-immunogenic and is therefore well tolerated by the patients.

Accordingly, a third object of the invention is a pharmaceutically active molecule, as described above, for use in the treatment of ocular pathologies, preferably pathologies of the front of the eye, wherein said pharmaceutically active molecule is administered in a drug delivery system according to the first object of the invention.

A method for the treatment of ocular pathologies, preferably pathologies of the front of the eye, comprising administering a pharmaceutically active molecule in a drug delivery system according to the first object of the invention is disclosed but not part of the invention.

### EXPERIMENTAL SECTION

### Example 1:

### Preparation of microparticles loaded with rhNGF

Microparticles formed by a polylactic co-glycolic acid (PLGA) matrix loaded with rhNGF were prepared according to the procedure described below.

A 0.85 mg/mL solution of rhNGF in Formulation Buffer (50 mM Sodium phosphate monobasic monohydrate, 100 mM Sodium Chloride, pH 7.2) was prepared.

An aqueous solution W1 was prepared containing 150 µL of the rhNGF solution prepared above, 6.01 mg human serum albumin (HSA) and 150 µL of polyethylene glycol 400 (PEG400) (ratio v/w/v of 1:40:1).

150 µL of solution W1 were then added to 7.5 mL of a solution O containing 20% w/v of PLGA (Resomer^{®} RG752H) in ethyl acetate and homogenized at 17400 rpm for 3 minutes, in by a homogenizer Ultraturrax t25 basic (IKA), using 8G probe.

The obtained emulsion W1/O was quickly added to 30 mL aqueous solution W2, containing 1% w/v of polyvinyl alcohol (PVA) (Mowiol^{®} 40-88) doped with 900 µL of ethyl acetate and homogenized again at 12000 rpm for 30 minutes. The resulting double emulsion (W1/O/W2), water down to wash the tube and recover material, was stirred for 3 hours at 500 rpm, at room temperature using overhead stirrer RW20D (IKA), with a PTFE-coated-2-bladed propeller stirrer, to allow organic solvent evaporation and the precipitation of microparticles. The suspension was collected in two 50 mL polypropylene tubes (Eppendorf Lobind protein) and centrifuged at 7400 rpm, for 15 minutes, at 4 °C. The microparticles were isolated and washed 6 times using 50 mL milliQ water, by centrifugation at 7000 rpm for 15 minutes at 4°C. Afterwards, the microparticles were re-suspended in aqueous solution of trehalose 2% w/v. The obtained suspension was moved in ISO glass type siliconized Class 1 vials and freezed at -80 °C to be, subsequently, lyophilized. The microparticles produced using this procedure show an encapsulation efficiency of more than 73% (55.5 ng rhNGF/mg microparticles vs theoretical value of 76 ng rhNGF/mg microparticles), with a particle size distribution between 1.9 and 6.9 µm, as measured with the Hydro 2000µP wet sample dispersion method using the analyzer Mastersizer2000 (Malvern). The final composition of the microparticles comprises 89% w/w of PLGA.

### Example 2:

### Preparation of dehydrated, decellularized lenticles

Human corneal lenticules having a thickness between 100 and 150 µm were obtained from SMILE surgery (SMall Incision Lenticule Extraction), according to the procedure described in Sekundo W, Kunert KS, Blum M. Br J Ophthalmol. 2011; 95:335-339, and cryopreserved until further processing.

Later, the lenticules were prepared to be used for functionalization according to the following procedure.

The lenticules were thawed and the cell component of the lenticules was eliminated by incubation in SDS 0.1% solution for 24 hours at R.T. followed by 3 washes in PBS (24 hours each).

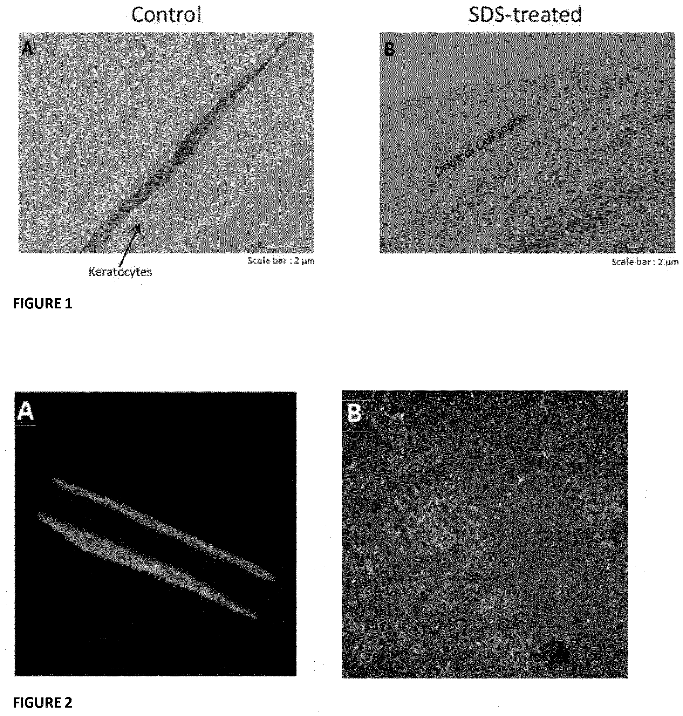

As shown in Figure 1, which shows the analysis by Transmission Electronic Microscopy (TEM) of a lenticule before (panel A) and after (panel B) treatment with the SDS solution, a complete removal of the cell component was obtained.

The decellularized corneal lenticules were then dehydrated by treatment at 60 °C for 2 hours and stored at room temperature until use.

### Example 3:

### Functionalization of lenticules

The microparticles loaded with rhNGF prepared in Example 1 were used to functionalize the dehydrated, decellularized lenticules obtained in Example 2.

In details, 20 mg of microparticles obtained in Example 1 were suspended in 0.175 mL of 0.9% NaCl and one lenticule was added to the obtained suspension and incubated for 3 hours at R.T. with orbital shaking at 200 rpm.

The lenticule was then removed from the suspension and washed 10 times in 0.4 mL of 0.9% NaCl (up&down).

### Example 4

### Analysis of functionalized lenticules

A functionalized lenticule prepared as described in Example 3 was lysed by incubation with 0.2 mL of 5 mg/mL collagenase IA in PBS containing 100 mg/L Ca^{2+/}Mg²⁺ at 37 °C for 30 minutes, this step was carried out to specifically lyse the lenticule preserving the already released rhNGF (supernatant) and the whole rhNGF-MPs (pellet). The lysed samples were centrifuged for 10 minutes at 8000 rpm at 4 °C.

The supernatant phase obtained by centrifugation contains the rhNGF already released by the microparticles whereas the pellet phase contains intact MPs.

The supernatant phase was collected and immediately stored it at -20 ° until analysis. The remaining pellet, corresponding to intact microparticles released by the lenticule upon lysis, was treated with 0.05 mL of Ethyl Acetate to lyse the PLGA shell of MPs and 0.100 mL of PBS containing 100 mg/L Ca^{2+/}Mg²⁺ was added to safely extract the rhNGF. The samples were centrifuged for 5 minutes at 8000 rpm and the aqueous phase collected (Pellet phase).

The Supernatant and Pellet phase were each diluted 1:3 in PBS containing 100 mg/L Ca^{2+/}Mg²⁺ and analyzed by ELISA assay to detect the concentration of rhNGF.

The supernatant phase contains the rhNGF already released by the MPs whereas the pellet phase contains intact MPs.

The results obtained from the analysis of three lenticules are shown in Table 1 below

**Table 1:**

| Sample | | Concentration rhNGF in each phase (pg/mL) | Total concentratio n of rhNGF (pg/mL) | rhNGF free&released/ rhNGF in MP |
|---|---|---|---|---|
| Lenticule 1 | Supernatant (free&released rhNGF) | 6387 | 7617 | 84% |
| | Pellet (intact MPs) | 1230 | | 16% |
| Lenticule 2 | Supernatant (free&released rhNGF) | 3455 | 4384 | 79% |
| | Pellet (intact MPs) | 929 | | 21% |
| Lenticule 3 | Supernatant (free&released rhNGF) | 3904 | 4639 | 84% |
| | Pellet (intact MPs) | 735 | | 16% |

The results obtained demonstrate that the ratio between the rhNGF released and that contained in the microparticles is highly reproducible in the different lenticules.

### Example 5:

### Distribution of rhNGF-MPs in lenticules

Fluorescent PLGA microparticles (Fluo-MPs) (Supplied by S.I.C. #LGFG5000) were used to test the interaction of the microparticles with the lenticule. 5 mg of Fluo-MPs were suspended in 0.175 mL of 0.9% and one lenticule was added to the obtained suspension and incubated for 3 hours at R.T with orbital shaking at 200 rpm.

The lenticule was then removed from the suspension and washed 10 times in 0.4 mL of 0.9% NaCl (up&down).

In order to evaluate the binding to or inclusion of the Fluo-MPs in the lenticule and their location, the collagen in the lenticule was stained by immunofluorescence using the protocol reported below.

The lenticule was fixed in paraformaldehyde 4% for 10 min, washed 3 times in PBS 1X for 5 min and then incubated for 1 hour with Anti-Collagen I primary antibody (Abcam, Cat# ab34710) in BSA 1% prepared in PBS 1X. It was then washed again 3 times in PBS 1X for 5 min and incubated for 1 hour with Anti-rabbit Cy3 secondary antibody (Jackson ImmunoReasearch, Laboratories, Cat# 111-165-144)) in BSA 1% prepared in PBS 1X and washed 3 times in PBS 1X for 5 min.

The immunostained lenticule was finally mounted on coverslip and analysed by LMS-800 Zeiss confocal microscope.

The data obtained, shown in Figure 2, demonstrate that the microparticles are mainly bound at the surface of lenticule.

### Example 6

### In vitro kinetic release of rhNGF from MPs included in lenticule

The in vitro release kinetic of rhNGF from the functionalized lenticules was evaluated with the following procedure.

A functionalized lenticule obtained as described in Example 3 was incubated in a tube containing 0.150 mL of PBS at 37 °C with 80 rpm orbital shaking (LoBind tubes were used to preserve rhNGF)

Time points were set at t=0, 2, 4 or 24 hours and t=2, 5, 7, 9, 12, 14, 16, 19, 21, 23 or 25 days.

At every time point, the lenticule was removed from the initial tube and moved to the new tube containing 0.150 mL of PBS and incubated again at 37 °C with 80 rpm orbital shaking.

Each sample was immediately stored at -20 °C;
After 1 month the samples were analyzed, without dilution, by ELISA assay to evaluate the rhNGF title.

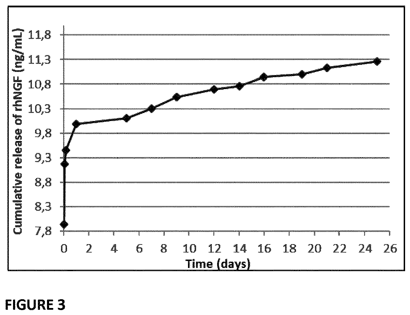

As shown in Figure 3, the rhNGF release from MPs included in the lenticule is sustained for about one month. Particularly, the neurotrophin is quickly released in the first 24 hours and the release is constantly maintained up to the end of the experiment.

## Claims

1. A drug delivery system comprising a decellularized corneal stroma scaffold having dispersed within and/or bound to the surface microparticles containing at least one pharmaceutically active molecule dispersed in a matrix having a composition comprising at least 70% w/w of polylactic co-glycolic acid (PLGA).

2. A drug delivery system according to claim 1, wherein said corneal stroma scaffold is cell-free.

3. A drug delivery system according to claim 1 or 2, wherein said matrix comprises at least 75% (w/w), preferably 80% (w/w), more preferably 85% (w/w) of polylactic co-glycolic acid (PLGA) and/or said matrix comprises between 85% (w/w) and 95% (w/w), more preferably 89% (w/w), of polylactic co-glycolic acid (PLGA).

4. A drug delivery system according to claims 1 or 3, wherein said matrix further contains polyethylene glycol, preferably low-molecular-weight grade polyethylene glycol, more preferably PEG400, albumin, preferably human serum albumin (HSA), and/or Trehalose.

5. A drug delivery system according to claims 1 to 4, wherein said microparticles have a particle size distribution measured by laser diffraction technology between 1 and 15 µm, preferably between 1.5 and 10 µm , more preferably between 1.9 and 7 µm.

6. A drug delivery system according to claims 1 to 5, wherein said corneal stroma scaffold is a graft obtained from a donor cornea.

7. A drug delivery system according to claims 1 to 6, wherein said corneal stroma scaffold is a corneal stroma lenticule.

8. A drug delivery system according to claim 7, wherein said corneal stroma lenticule is obtained from a donor cornea by refractive eye surgery, preferably refractive lenticule extraction (ReLEx) or femtosecond lenticule extraction (FLEx).

9. A drug delivery system according to claim 7 or 8, wherein said corneal stroma lenticule has a thickness between 80 and 300 µm, preferably between 100 and 150 µm and/or a diameter between 4 and 9 mm, preferably between 5 and 8 mm, more preferably between 6 and 7 mm.

10. A drug delivery system according to claims 1 to 9, wherein said pharmaceutically active molecule is a protein or a peptide.

11. A drug delivery system according to claim 10, wherein said pharmaceutically active molecule is a growth factor or a growth factor-mimetic peptide or protein.

12. A drug delivery system according to claim 10 or 11, wherein said pharmaceutically active molecule is a neurotrophin or a neurotrophin-mimetic peptide or protein.

13. A drug delivery system according to claims 10 to 12, wherein said pharmaceutically active molecule is nerve growth factor or a nerve growth factor-mimetic peptide or protein.

14. A drug delivery system according to claim 13, wherein the amount of said nerve growth factor in said microparticles is between 30 and 100 ng, preferably between 50 and 90 ng, more preferably between 60 and 80 ng for each mg of microparticles.

15. Method for preparing a drug delivery system as claimed in claims 1 to 14, comprising the following steps:
a. obtaining a corneal stroma scaffold, preferably a corneal stroma lenticule from a donor cornea;
b. removing cellular material from said scaffold, if present, thus obtaining a decellularized corneal stroma scaffold;
c. incubating said decellularized corneal stroma scaffold in a suspension of microparticles containing at least one pharmaceutically active molecule dispersed in a matrix having a composition comprising at least 70% (w/w) of polylactic co-glycolic acid (PLGA).

16. Method as claimed in claim 15, wherein in step b. said cellular material is removed by incubating the corneal stroma scaffold in a solution of a surfactant.

17. Method as claimed in claim 16, wherein said surfactant is Sodium Dodecyl Sulfate, preferably at a concentration between 0.05 and 0.2%, preferably 0.1%.

18. Method as claimed in claims 15 to 17, wherein said microparticles of step c. are obtained by the following steps:
a. preparing a water/oil emulsion containing the pharmaceutically active principle and polylactic co-glycolic acid.
b. preparing an aqueous solution containing polyvinyl alcohol doped with the same organic solvent used in the oil phase of step a.
c. mixing the emulsion and solution prepared in a. and in b. and homogenizing, thus obtaining a water/oil/water emulsion.
d. evaporating the organic solvent, thus obtaining precipitation of microparticles;
e. recovering and washing the precipitated microparticles;
f. freeze-drying the microparticles.

19. A pharmaceutically active molecule for use in the treatment of ocular pathologies, preferably pathologies of the front of the eye, wherein said pharmaceutically active molecule is administered in a drug delivery system according to claim 1 to 14.

## Patentansprüche

1. Arzneimittelabgabesystem, umfassend ein dezellularisiertes Hornhautstroma-Gerüst mit darin dispergierten und/oder an die Oberfläche gebundenen Mikropartikeln, die mindestens ein pharmazeutisch wirksames Molekül enthalten, das in einer Matrix mit einer Zusammensetzung dispergiert ist, die mindestens 70 % Gew./Gew. Polymilch-Co-Glykolsäure (PLGA) umfasst.

2. Arzneimittelabgabesystem nach Anspruch 1, wobei das Hornhautstroma-Gerüst zellfrei ist.

3. Arzneimittelabgabesystem nach Anspruch 1 oder 2, wobei die Matrix mindestens 75 % (Gew./Gew.), vorzugsweise 80 % (Gew./Gew.), bevorzugter 85 % (Gew./Gew.) Polymilch-Co-Glykolsäure (PLGA) umfasst und/oder die Matrix zwischen 85 % (Gew./Gew.) und 95 % (Gew./Gew.), bevorzugter 89 % (Gew./Gew.), Polymilch-Co-Glykolsäure (PLGA) umfasst.

4. Arzneimittelabgabesystem nach Anspruch 1 oder 3, wobei die Matrix ferner Polyethylenglykol, vorzugsweise Polyethylenglykol mit niedrigem Molekulargewicht, bevorzugter PEG400, Albumin, vorzugsweise Humanserumalbumin (HSA), und/oder Trehalose enthält.

5. Arzneimittelabgabesystem nach den Ansprüchen 1 bis 4, wobei die Mikropartikel eine mittels Laserbeugungstechnik gemessene Partikelgrößenverteilung zwischen 1 und 15 µm, vorzugsweise zwischen 1,5 und 10 µm, bevorzugt zwischen 1,9 und 7 µm aufweisen.

6. Arzneimittelabgabesystem nach den Ansprüchen 1 bis 5, wobei das Hornhautstroma-Gerüst ein Transplantat ist, das aus einer Spenderhornhaut gewonnen wurde.

7. Arzneimittelabgabesystem nach den Ansprüchen 1 bis 6, wobei das Hornhautstroma-Gerüst ein Hornhautstroma-Lentikel ist.

8. Arzneimittelabgabesystem nach Anspruch 7, wobei das Hornhautstroma-Lentikel aus einer Spenderhornhaut durch refraktive Augenchirurgie, vorzugsweise refraktive Lentikelextraktion (ReLEx) oder Femtosekunden-Lentikelextraktion (FLEx), gewonnen wird.

9. Arzneimittelabgabesystem nach Anspruch 7 oder 8, wobei das Hornhautstroma-Lentikel eine Dicke zwischen 80 und 300 µm, vorzugsweise zwischen 100 und 150 µm und/oder einen Durchmesser zwischen 4 und 9 mm, vorzugsweise zwischen 5 und 8 mm, bevorzugt zwischen 6 und 7 mm aufweist.

10. Arzneimittelabgabesystem nach den Ansprüchen 1 bis 9, wobei das pharmazeutisch aktive Molekül ein Protein oder ein Peptid ist.

11. Arzneimittelabgabesystem nach Anspruch 10, wobei das pharmazeutisch aktive Molekül ein Wachstumsfaktor oder ein wachstumsfaktormimetisches Peptid oder Protein ist.

12. Arzneimittelabgabesystem nach Anspruch 10 oder 11, wobei das pharmazeutisch aktive Molekül ein Neurotrophin oder ein neurotrophinmimetisches Peptid oder Protein ist.

13. Arzneimittelabgabesystem nach den Ansprüchen 10 bis 12, wobei das pharmazeutisch aktive Molekül ein Nervenwachstumsfaktor oder ein nervenwachstumsfaktormimetisches Peptid oder Protein ist.

14. Arzneimittelabgabesystem nach Anspruch 13, wobei die Menge des Nervenwachstumsfaktors in den Mikropartikeln zwischen 30 und 100 ng, vorzugsweise zwischen 50 und 90 ng, bevorzugter zwischen 60 und 80 ng pro mg Mikropartikel beträgt.

15. Verfahren zur Herstellung eines Arzneimittelabgabesystems nach den Ansprüchen 1 bis 14, umfassend die folgenden Schritte:
a. Gewinnen eines Hornhautstroma-Gerüsts, vorzugsweise eines Hornhautstroma-Lentikels aus einer Spenderhornhaut;
b. Entfernen von zellulärem Material von dem Gerüst, falls vorhanden, wodurch ein dezellularisiertes Hornhautstroma-Gerüst erhalten wird;
c. Inkubieren des dezellularisierten Hornhautstroma-Gerüsts in einer Suspension von Mikropartikeln, die mindestens ein pharmazeutisch aktives Molekül enthalten, das in einer Matrix mit einer Zusammensetzung, umfassend mindestens 70 % (Gew./Gew.) Polymilch-Co-Glykolsäure (PLGA) dispergiert ist.

16. Verfahren nach Anspruch 15, wobei in Schritt b. das zelluläre Material durch Inkubieren des Hornhautstroma-Gerüsts in einer Lösung eines Tensids entfernt wird.

17. Verfahren nach Anspruch 16, wobei das Tensid Natriumdodecylsulfat ist, vorzugsweise in einer Konzentration zwischen 0,05 und 0,2%, vorzugsweise 0,1%.

18. Verfahren nach den Ansprüchen 15 bis 17, wobei die Mikropartikel aus Schritt c. durch die folgenden Schritte erhalten werden:
a. Herstellen einer Wasser/Öl-Emulsion, die den pharmazeutischen Wirkstoff und Polymilch-Co-Glykolsäure enthält.
b. Herstellen einer wässrigen Lösung, die Polyvinylalkohol enthält, der mit demselben organischen Lösungsmittel dotiert ist, das in der Ölphase von Schritt a verwendet wurde.
c. Mischen der unter a. und unter b. hergestellten Emulsion und Lösung und Homogenisieren, wodurch eine Wasser/Öl/Wasser-Emulsion erhalten wird.
d. Verdampfen des organischen Lösungsmittels, wodurch eine Ausfällung von Mikropartikeln erhalten wird;
e. Wiederherstellen und Waschen der ausgefällten Mikropartikel;
f. Gefriertrocknen der Mikropartikel.

19. Pharmazeutisch aktives Molekül zur Verwendung bei der Behandlung von Augenpathologien, vorzugsweise Pathologien der Vorderseite des Auges, wobei das pharmazeutisch aktive Molekül in einem Arzneimittelabgabesystem nach Anspruch 1 bis 14 verabreicht wird.

## Revendications

1. Système d'administration de médicament comprenant un échafaudage de stroma cornéen décellularisé ayant, dispersées en son sein et/ou liées à la surface, des microparticules contenant au moins une molécule pharmaceutiquement active dispersée dans une matrice ayant une composition comprenant au moins 70 % p/p de poly(acide lactique co-glycolique) (PLGA).

2. Système d'administration de médicament selon la revendication 1, dans lequel ledit échafaudage de stroma cornéen est dépourvu de cellules.

3. Système d'administration de médicament selon la revendication 1 ou 2, dans lequel ladite matrice comprend au moins 75 % (p/p), de préférence 80 % (p/p), plus préférentiellement 85 % (p/p) de poly(acide lactique co-glycolique) (PLGA) et/ou ladite matrice comprend entre 85 % (p/p) et 95 % (p/p), plus préférentiellement 89 % (p/p), de poly(acide lactique co-glycolique) (PLGA).

4. Système d'administration de médicament selon les revendications 1 ou 3, dans lequel ladite matrice contient en outre du polyéthylène glycol, de préférence du polyéthylène glycol à faible poids moléculaire, plus préférentiellement du PEG400, de l'albumine, de préférence de l'albumine sérique humaine (HSA), et/ou du tréhalose.

5. Système d'administration de médicament selon les revendications 1 à 4, dans lequel lesdites microparticules possèdent une granulométrie mesurée par une technologie de diffraction laser entre 1 et 15 µm, de préférence entre 1,5 et 10 µm, plus préférentiellement entre 1,9 et 7 µm.

6. Système d'administration de médicament selon les revendications 1 à 5, dans lequel ledit échafaudage de stroma cornéen est un greffon obtenu à partir de la cornée d'un donneur.

7. Système d'administration de médicament selon les revendications 1 à 6, dans lequel ledit échafaudage de stroma cornéen est un lenticule de stroma cornéen.

8. Système d'administration de médicament selon la revendication 7, dans lequel ledit lenticule de stroma cornéen est obtenu à partir de la cornée d'un donneur par chirurgie oculaire réfractive, de préférence par extraction lenticulaire réfractive (ReLEx) ou par extraction lenticulaire femtoseconde (FLEx).

9. Système d'administration de médicament selon la revendication 7 ou 8, dans lequel ledit lenticule de stroma cornéen possède une épaisseur entre 80 et 300 µm, de préférence entre 100 et 150 µm et/ou un diamètre entre 4 et 9 mm, de préférence entre 5 et 8 mm, plus préférentiellement entre 6 et 7 mm.

10. Système d'administration de médicament selon les revendications 1 à 9, dans lequel ladite molécule pharmaceutiquement active est une protéine ou un peptide.

11. Système d'administration de médicament selon la revendication 10, dans lequel ladite molécule pharmaceutiquement active est un facteur de croissance ou un peptide ou une protéine imitant un facteur de croissance.

12. Système d'administration de médicament selon la revendication 10 ou 11, dans lequel ladite molécule pharmaceutiquement active est une neurotrophine ou un peptide ou une protéine imitant une neurotrophine.

13. Système d'administration de médicament selon les revendications 10 à 12, dans lequel ladite molécule pharmaceutiquement active est un facteur de croissance nerveuse ou un peptide ou une protéine imitant un facteur de croissance nerveuse.

14. Système d'administration de médicament selon la revendication 13, dans lequel la quantité dudit facteur de croissance nerveuse dans lesdites microparticules est entre 30 et 100 ng, de préférence entre 50 et 90 ng, plus préférentiellement entre 60 et 80 ng pour chaque mg de microparticules.

15. Procédé pour la préparation d'un système d'administration de médicament selon les revendications 1 à 14, comprenant les étapes suivantes :
a. l'obtention d'un échafaudage de stroma cornéen, de préférence d'un lenticule de stroma cornéen provenant de la cornée d'un donneur ;
b. le retrait de matériel cellulaire dudit échafaudage, si présent, ce qui permet d'obtenir un échafaudage de stroma cornéen décellularisé ;
c. l'incubation dudit échafaudage de stroma cornéen décellularisé dans une suspension de microparticules contenant au moins une molécule pharmaceutiquement active dispersée dans une matrice ayant une composition comprenant au moins 70 % (p/p) de poly(acide lactique co-glycolique) (PLGA).

16. Procédé selon la revendication 15, dans lequel, à l'étape b., ledit matériel cellulaire est retiré par l'incubation de l'échafaudage de stroma cornéen dans une solution d'un tensioactif.

17. Procédé selon la revendication 16, dans lequel ledit tensioactif est le dodécylsulfate de sodium, de préférence à une concentration entre 0,05 et 0,2 %, de préférence de 0,1 %.

18. Procédé selon les revendications 15 à 17, dans lequel lesdites microparticules de l'étape c. sont obtenues par les étapes suivantes :
a. la préparation d'une émulsion eau/huile contenant le principe pharmaceutiquement actif et du poly(acide lactique co-glycolique).
b. la préparation d'une solution aqueuse contenant du poly(alcool de vinyle) dopé avec le même solvant organique que celui utilisé dans la phase huileuse de l'étape a.
c. le mélange de l'émulsion et de la solution préparées en a. et en b. et leur homogénéisation, ce qui permet d'obtenir une émulsion eau/huile/eau.
d. l'évaporation du solvant organique, ce qui permet d'obtenir la précipitation de microparticules ;
e. la récupération et le lavage des microparticules précipitées ;
f. la lyophilisation des microparticules.

19. Molécule pharmaceutiquement active pour une utilisation dans le traitement de pathologies oculaires, de préférence de pathologies de la partie antérieure de l'œil, dans laquelle ladite molécule pharmaceutiquement active est administrée dans un système d'administration de médicament selon les revendications 1 à 14.
